(19) Europäisches Patentamt
European Patent Office
Office européen des brevets

(11) Numéro de publication: **0 157 679**
**B1**

(12) # FASCICULE DE BREVET EUROPÉEN

(45) Date de publication du fascicule du brevet: **18.05.88**

(51) Int. Cl.⁴: **C 09 K 19/20**, C 07 C 51/58

(21) Numéro de dépôt: **85400476.9**

(22) Date de dépôt: **12.03.85**

(54) **Composé organique présentant une phase smectique A, mélange comprenant ce composé et procédé de fabrication.**

(30) Priorité: **16.03.84 FR 8404109**

(43) Date de publication de la demande: **09.10.85 Bulletin 85/41**

(45) Mention de la délivrance du brevet: **18.05.88 Bulletin 88/20**

(84) Etats contractants désignés: **DE**

(56) Documents cité: **EP-A-0 011 002**

(73) Titulaire: **THOMSON- CSF, 173, Boulevard Haussmann, F-75379 Paris Cédex 08 (FR)**

(72) Inventeur: **Le Barny, Pierre, THOMSON- CSF SCPI 173, bld Haussmann, F-75379 Paris Cedex 08 (FR)**
Inventeur: **Dubois, Jean- Claude, THOMSON- CSF SCPI 173, bld Haussmann, F-75379 Paris Cedex 08 (FR)**
Inventeur: **Ravaux, Gilles, THOMSON- CSF SCPI 173, bld Haussmann, F-75379 Paris Cedex 08 (FR)**

(74) Mandataire: **Lepercque, Jean, THOMSON- CSF SCPI 19, avenue de Messine, F-75008 Paris (FR)**

**EP 0 157 679 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1988

## Description

L'invention concerne une famille de composés organiques du type ester de 1-(4 hydroxyphényl) 2(4'trifiuorométhylphényl) éthane qui présente une mésophase de type smectique A et éventuellement une mésophase nématique. L'invention concerne également le procédé de fabrication des molécules de cette famille, ainsi que les mélanges obtenus à partir de ces esters et possédant également une phase smectique A.

On connaît, par EP-A-11 002, une famille de composés organiques mésomorphes d'un type voisin mais ne presentant en particulier aucun pont du genre 1,2-diphényl-éthane.

Les composés selon l'invention possèdent la propriéte, lorsqu'on les mélange à des cristaux liquides smectiques A, d'élargir la plage smectique que présentaient ces produits. Ceci est un avantage lorsque de tels mélanges sont utilisés dans des dispositifs de visualisation qui voient leur gamme d'utilisation s'élargir à la fois vers les hautes et vers les basses températures.

Les composes selon l'invention répondent à la formule chimique générale:

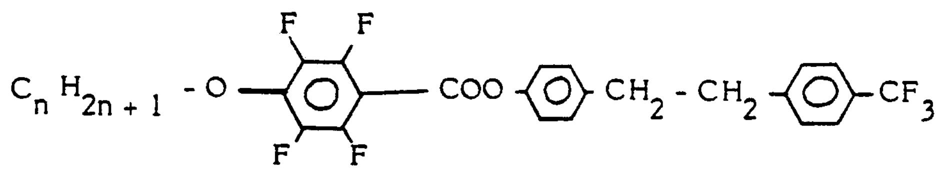

avec $1 \leqslant n \leqslant 15$.

Les composés répondant à cette formule peuvent être définis par la dénomination suivante : 1-(4 alkyloxyfluorobenzoyloxyphényl) 2-(4'trifluorométhylphényl) éthane.

L'invention a donc pour objet un composé organique présentant au moins une phase mésomorphe de type smectique A, caractérisé en ce qu'il répond à la formule chimique générale :

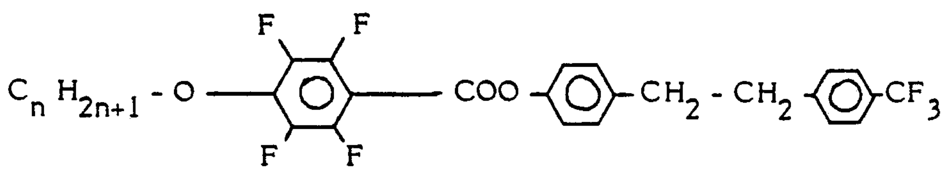

pour laquelle $1 \leqslant n \leqslant 15$.

L'invention a aussi pour objet un mélange de cristaux liquides présentant au moins une phase smectique A, caractérisé en ce qu'il comporte au moins l'un des composés organiques cités ci-dessus.

L'invention a encore pour objet un procédé de fabrication d'un composé organique tel que cité ci-dessus, caractérisé en ce que ledit composé est un produit de la réaction du chlorure d'acide $C_nH_{2n+1}$

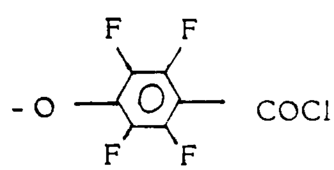

et du phénol 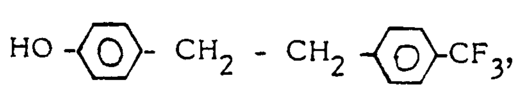

ladite réaction s'effectuant à température ambiante dans la pyridine.

L'invention sera mieux comprise et d'autres avantages apparaîtront au moyen de la description qui va suivre et de la figure annexée qui est un diagram me de phase isobare.

La description qui va suivre portera sur le procédé général de synthèse des molécules selon l'invention, ainsi que sur les propriétés mésophormes des cristaux liquides correspondants. De plus, un exemple d'incorporation d'un composé selon l'invention dans un matériau smectique A destiné à un affichage utilisant un effet mixte thermique et électrique, mettra en évidence l'élargissement de la gamme d'utilisation de cet affichage vers les basses et les hautes températures.

**PROCEDE GENERAL DE SYNTHESE**

Les composés organiques selon l'invention peuvent être obtenus en 8 étapes à partir de produits de base tels que : l'acide 4 bromobenzoïque, le méthoxybenzène, l'acide pentafluorobenzoïque et selon le schéma réactionnel suivant.

Réaction 1: Obtention du chloruore de 4 bromophénylacétyl. Ce chlorure d'acide est obtenu par action du chlorure de thionyle sur l'acide 4 bromophénylacétique.

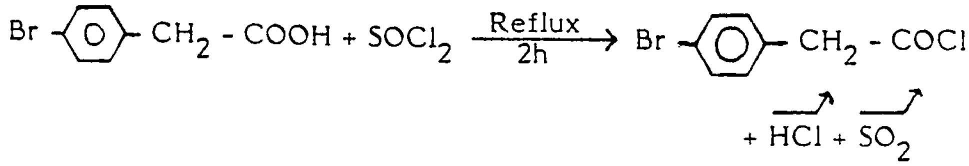


Réaction 2: Synthèse du 4-(4 bromophénylacétyl) 4'-méthoxyphényl. Le 4-(4 bromophénylacétyl) 4'-méthoxyphényl est obtenu par action du chlorure de 4 bromophénylacétyl sur le méthoxybenzène en présence de chlorure d'aluminium et de chlorure de méthylène, à une température de 5°. (Réaction de FRIEDEL-CRAFT).

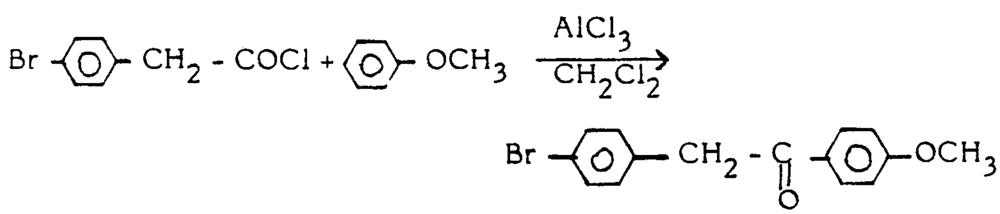


Réaction 3: Obtention du 1-(4 bromophényl) 2-(4'méthoxyphényl) éthane. Le 4-(4 bromophénylacéyl) 4'-méthoxyphényl est réduit en 1-(4 bromophényl) 2-(4'méthoxyphényl) éthane selon la réaction de WOLF-KISHNER modifiée par HUANG-MINLON, par action de l'hydrazine en milieu basique et en présence de diéthylène glycol. Le milieu basique est obtenu par de la potasse.

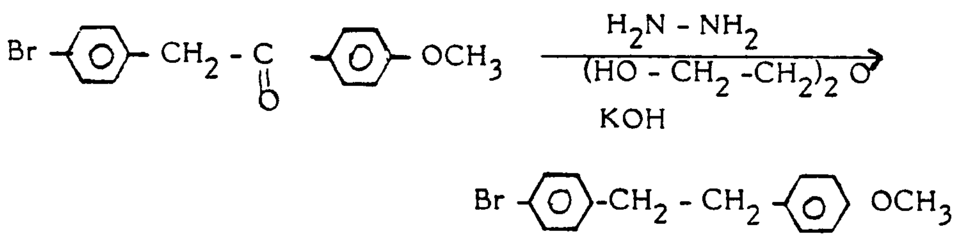


Réaction 4: Synthèse du 1(4 trifluorométhylphényl) 2-(4'-méthoxyphényl) éthane. Le 1-(4 trifluorométhylphényl) 2-(4'-méthoxyphényl) éthane est obtenu par trifluorométhylation du 1-(4 bromophényl) 2-(4'-méthoxyphényl) éthane en autoclave, à 150°C, en utilisant le diméthylformamide com me solvant, le iodotrifluorométhane comme agent de trifluorométhylation et le cuivre en poudre com me agent de couplage.

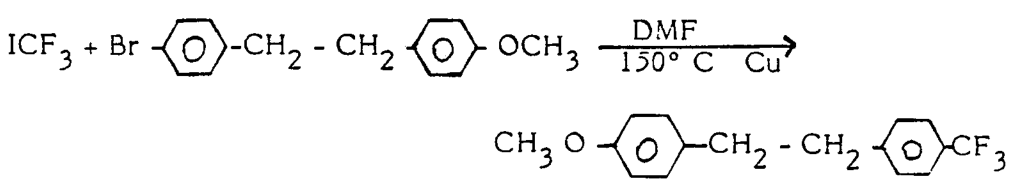


Réaction 5: Synthèse du 1-(4 trifluorométhylphényl) 2-(4'-hydroxyphényl) éthane. Ce phénol est obtenu par déméthylation du 1-(4 trifluorométhylphényl 2-(4'-méthoxyphényl) éthane par le iodotriméthylsilane a température ambiante, en présence de chloroforme. La durée de la réaction est d'environ 5 jours.

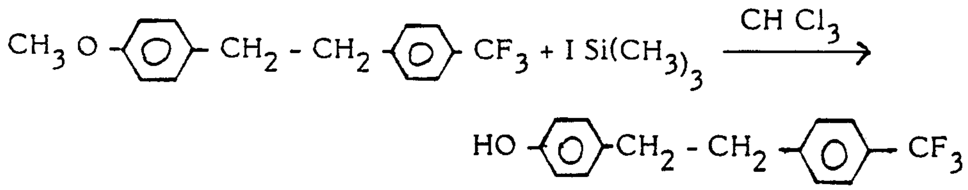


Réaction 6: Synthèse des acides 4 alkyloxyfluorobenzoïques. Les acides 4 alkyloxyfluorobenzoïques sont obtenus en une étape à partir de l'acide pentafluorobenzoïque par action de l'alcoolate de sodium sur le pentafluorobenzoate de sodium, le solvant étant l'alcool correspondant, puis en acidifiant le mélange réactionnel en fin de réaction. La réaction s'effectue à 60°C et dure 48 heures.

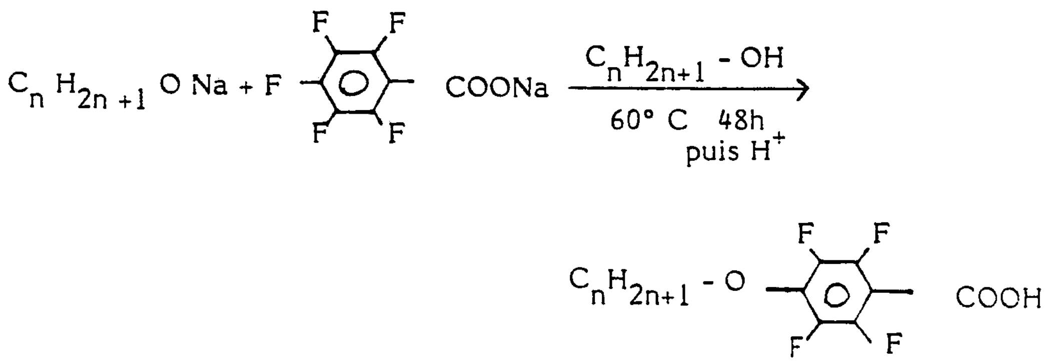


Réaction 7: Synthèse des chlorures de 4 alkyloxyfluorobenzoyle. Les chlorures d'acide sont obtenus par action du chlorure de thionyle sur les acides 4 alkoxyfluorobenzoïques.

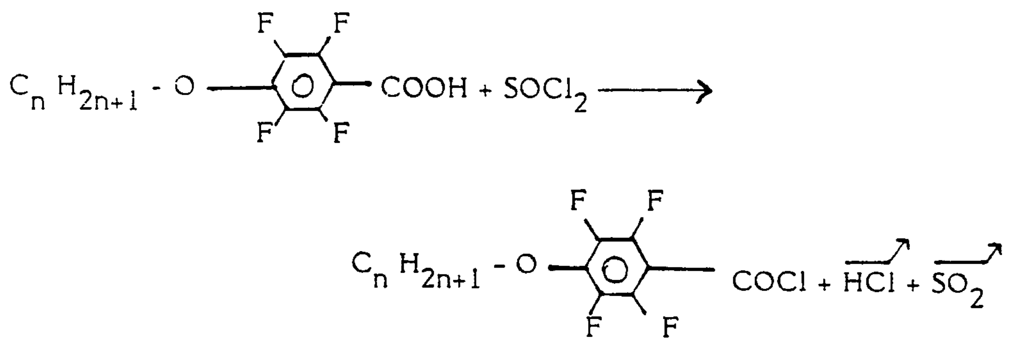

Réaction 8: Synthèse des (4 alkyloxyfluorobenzoyloxyphényl) 2-(4'trifluorométhylphényl) éthane. La synthèse de ces esters se fait en une étape à partir du chlorure d'acide $C_nH_{2n+1}$

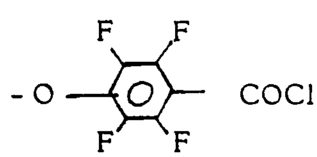

et du phénol 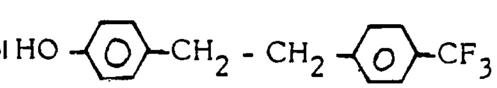

obtenu par la réaction 5. La réaction 8 s'effectue à température ambiante dans la pyridine.

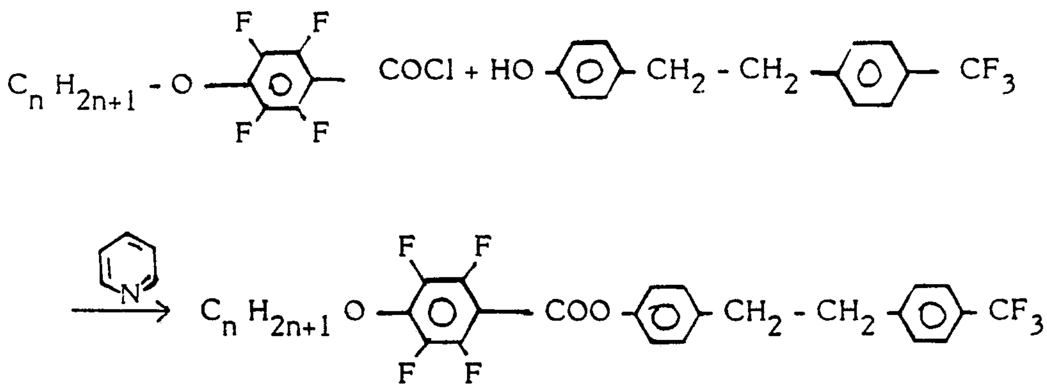

Les réactions 1, 2, 3, 7 et 8 sont classiques. La réaction 6 dérive d'un mode opératoire connu (voir BURDON, HOLLYHEAD, TATLOW, Journal of Chemical Society, p 6336, 1965) mais modifié selon l'enseignement apporté par la demande de brevet français de la Demanderesse portant le numéro d'enregistrement 82.22074 (EP-A-113 293). Cette modification a pour objet d'augmenter le rendement de la réaction. Les réactions 4 et 5 sont inspirées de modes opératoires connus selon les enseignements respectifs de KOBAYASHI, KOMADAKI, SATO, HARA, CHIHAMI (Chemistry and Pharmacy Bulletin n° 18 (11), p 2334 à 2339, 1970) et JUNG, LYSTER (Journal of Organic Chemistry n° 42 (23), p 3761 à 3764, 1973), ces modes opératoires étant cependant adaptés à l'élaboration des composés selon l'invention.

**MODE OPERATOIRE DES REACTIONS 4 ET 5:**

1 - Réaction 4 : trifluorométhylation.

Dans le réacteur de 250 ml de l'autoclave, on introduit 70 ml de diméthylformamide (distillé sur du sulfate de cuivre Cu $SO_4$), 12,52 g ($4,3.10^{-2}$ mole) de 1-(4 bromophényl) 2-(4'-méthoxyphényl) éthane et 30 g de cuivre en poudre. Le réacteur est refroidi à -40°C et on introduit rapidement 100 g d'iodotrifluorométhane liquéfié. Lorsque le réacteur est revenu à la température ambiante, on le place dans le four de l'autoclave et on le chauffe à 150°C. On laisse la réaction se poursuivre à la même température pendant 20 heures, sous agitation.

Le milieu réactionnel refroidi est versé dans 400 ml d'eau. Le cuivre et ses sels sont éliminés par filtration sur du verre fritté n° 4. La fraction insoluble est lavée au chloroforme. Le filtrat est ensuite placé dans une ampoule à décanter. La phase aqueuse est éliminée. La phase organique est lavée à l'eau puis séchée sur le sulfate de magnésium Mg $SO_4$, filtrée et évaporée à sec et sous vide.

On obtient une huile de couleur marron qui est lavée à l'eau afin d'éliminer le diméthylformamide résiduel. Un précipité apparaît que l'on dissout ensuite dans l'hexane. Le filtrat est évaporé à sec ce qui donne 12 g de produit coloré.

Le produit brut est purifié par chromatographie liquide sur de la silice, avec de l'hexane comme éluant, suivie d'une recristallisation dans l'éthanol. On obtient finalement 2,5 g de 1-(4 trifluorométhylphényl) 2-(4'-méthoxyphényl) éthane dont le point de fusion est 80°C. Le rendement de la réaction est de 21 %.

2 - Réaction 5 : déméthylation.

Dans un erlenmeyer de 50 ml muni d'un agitateur magnétique, on dissout 2,51 g (c'est-à-dire 8,96 millimoles) de 1-(4 trifluorométhylphényl) 2-(4'-méthoxyphényl) éthane dans 23 ml de chloroforme distillé. On introduit ensuite rapidement 2,33 g (soit 11,64 millimoles) de iodotriméthylsilane. L'erlenmeyer est alors bouché hermétiquement et on laisse la réaction se poursuivre pendant 5 jours à la temperature ambiante. On ajoute ensuite 13 ml d'éthanol et l'agitation est maintenue pendant 1 heure. Puis on évapore les solvants sous vide.

Le produit brut est décoloré par un traitement au bisulfite de sodium, puis purifié par chromatographie liquide sur de la silice avec du toluène comme éluant. On obtient 1,89 g de phénol dont le point de fusion est de 126°C. Le rendement de la réaction est de 79,2 %.

Le mode opératoire qui vient d'être décrit est valable quelles que soient les valeurs de n pourvu que l'on respecte les proportions molaires.

**PROPRIETES DES CORPS SYNTHETISES:**

Le tableau ci-dessous donne, à titre d'exemple non limitatif, les résultats de l'étude calorimétrique effectuée sur deux des composés selon l'invention.

| N | n | K | | $S_A$ | | N | | I |
|---|---|---|---|---|---|---|---|---|
| 1 | 8 | X | 86,5 [5,76] | X | 90 [0,7] | X | 91 [0,5] | X |
| 2 | 11 | X | | | | | 89 [8,9] | X |
| | | | | X | | | (89) [1,2] | X |

Dans ce tableau, les lettres K, $S_A$, N et I désignent respectivement les phases cristalline smectique A, nématique et isotrope. Les croix situées sous ces lettres indiquent la transition d'une phase à une autre phase, lorsqu'elle s'effectue. Les températures de transition sont exprimées en degrés Celsius. Les valeurs entre crochets sont les enthalpies de transition exprimées en kcal/mole. La valeur entre parenthèses est une température correspondant à une phase monotropique.

Le composé n° 1, qui correspond à n = 8, présente une phase smectique A de 86,5°C à 90°C et une phase nématique de 90 à 91°C. Le composé n° 2 présente une mésophase $S_A$ métastable jusqu'à 66,5°C.

La nature des mésophases a été déterminée par l'étude de l'isomorphisme au microscope optique des composés étudiés avec des substances connues. A titre d'exemple, la figure annexée représente un diagramme de phase isobare obtenu par la mise en contact du composé n° 1 selon l'invention avec le produit du type ester de 2 hydroxyfluorène substitué de formule :

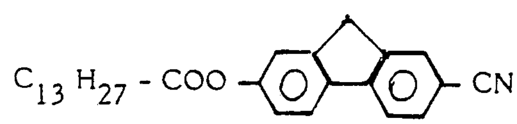

et qui possède la succession de phases suivante : K 76,4°C $S_A$ 91,5°C I. Cet ester est décrit dans la demande de brevet de la Demanderesse citée plus haut. Sur ce diagramme, l'axe des ordonnées de gauche (A) correspond à 100 % du composé n° 1 selon l'invention et l'axe des ordonnées de droite (B) correspond à 100 % de l'ester cité.

Il entre dans la cadre de l'invention d'utiliser les composés organiques selon l'invention comme cristaux liquides présentant une phase smectique A, seuls, en mélange entre eux ou avec d'autres cristaux liquides smectiques A afin den élargir le domaine d'application. A ce titre, ils peuvent être avantageusement utilisés dans des dispositifs de visualisation.

A titre d'exemple non limitatif, on va constater l'influence dans un mélange de cristaux liquides, de l'introduction d'un composé selon l'invention. Le 4 octyl 4' cyanobiphényle (produit C) de formule $C_8H_{17}$

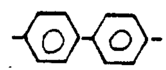

CN possède le diagramme de transitions de phases suivant :

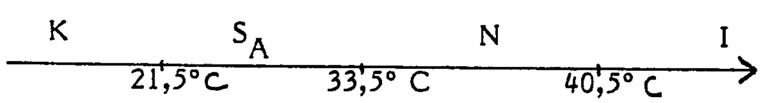


Le 4 décyl 4' cyanobiphényle (produit D) de formule $C_{19}H_{21}$

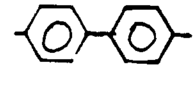

CN possède le diagramme de transitions de phases suivant:

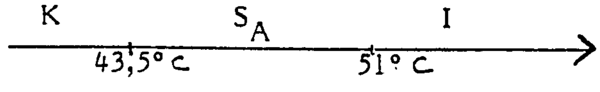


L'eutectique formé à partir des produits C et D possède un point de fusion de 13°C et un point de clarification de 43,3°C donc une plage mésomorphe (composée d'une phase smectique A et d'une phase nématique) qui s'étend sur 30,3°C. L'introduction du 1-(4 octyloxyfluorobenzoyloxyphényl) 2-(4'-trifluorométhylphényl) éthane, soit le composé numéro 1 du tableau, dans le mélange précédemment défini conduit à un eutectique ternaire comprenant 66 % de produit C, 22,6 % de produit D et 11,4 % du composé

numéro 1, qui possède un point de fusion de 10°C et une température de clarification de 48,6°C. La plage mésomorphe (composée d'une phase smectique A et d'une petite phase nématique) de ce mélange ternaire s'étend donc sur 38,6°C. Par rapport au mélange binaire décrit précédemment, on constate que la plage mésomorphe est plus étendue et que les températures limites de cette plage ont bien été repoussées vers le bas et vers le haut.

**Revendications**

1. Composé organique présentant au moins une phase mésomorphe de type smectique A, caractérisé en ce qu'il répond à la formule chimique générale:

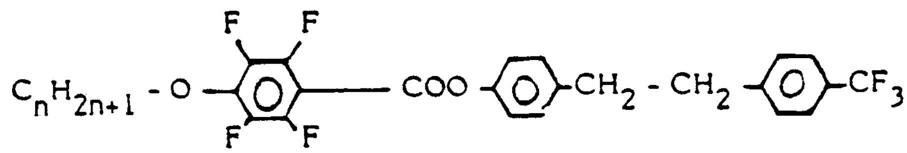

pour laquelle $1 \leqslant n \leqslant 15$.

2. Composé organique selon la revendication 1, caractérisé en ce que : $n = 8$.

3. Composé organique selon la revendication 1, caractérisé en ce que : $n = 11$.

4. Mélange de cristaux liquides présentant au moins une phase smectique A, caractérisé en ce qu'il comporte au moins un composé organique selon l'une quelconque des revendications 1 à 3.

5. Mélange selon la revendication 4, caractérisé en ce qu'il comprend 66 % de 4 octyl 4'-cyanobiphényle, 22,6 % de 4 décyl 4'-cyanobiphényle et 11,4 % dudit composé organique pour lequel $n = 8$.

6. Procédé de fabrication d'un composé organique selon l'une quelconque des revendications 1 à 3, caractérisé en ce que ledit composé est un produit de la réaction du chlorure d'acide $C_nH_{2n+1}$

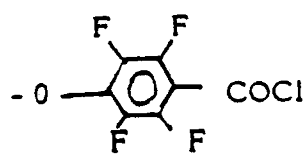

et du phénol 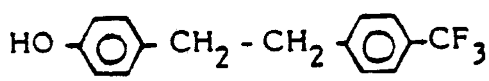

ladite réaction s'effectuant à température ambiante dans la pyridine.

7. Procédé de fabrication selon la revendication 6, caractérisé en ce qu'il comprend les étapes suivantes :

a) obtention du chlorure de 4 bromophénylacétyl par action du chlorure de thionyle sur l'acide 4 bromophénylacétique ;

b) synthèse du 4(4 bromophénylacétyl) 4'-méthoxyphényl par réaction de FRIEDEL-CRAFT ;

c) réduction du produit obtenu à l'étape précédente par une réaction de WOLF-KISHNER modifiée, par action de l'hydrazine en milieu basique et en présence de diéthylène glycol ;

d) trifluorométhylation du produit obtenu à l'étape c au moyen de l'iodotrifluorométhane en présence de cuivre à 150°C en autoclave ;

e) déméthylation du produit obtenu à l'étape d en utilisant le iodotriméthylsilane comme agent de clivage de la fonction éther méthylique.

**Patentansprüche**

1. Organische Verbindung mit mindestens einer mesomorphen Phase vom smektischen Typ A, dadurch gekennzeichnet, daß sie der allgemeinen chemischen Formel entspricht:

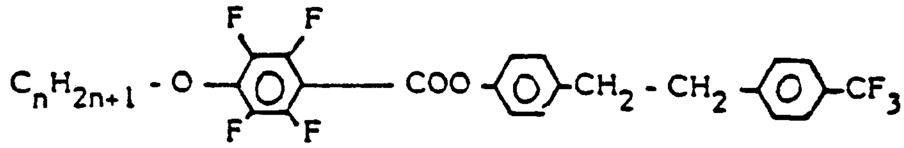

mit 1 ⩽ n ⩽ 15.

2. Organische Verbindung nach Anspruch 1, dadurch gekennzeichnet, daß n = 8.

3. Organische Verbindung nach Anspruch 1, dadurch gekennzeichnet, daß n = 11.

4. Mischung von Flüssigkristallen, die mindestens eine smektische Phase A aufweist, dadurch gekennzeichnet, daß sie mindestens eine organische Verbindung nach einem der Ansprüche 1 bis 3 enthält.

5. Mischung nach Anspruch 4, dadurch gekennzeichnet, daß sie 66 % 4-Octyl-4'-cyanobiphenyl, 22,6 % 4-Decyl-4'-cyanobiphenyl und 11,4 % der organischen Verbindung mit n = 8 enthält.

6. Verfahren zur Herstellung einer organischen Verbindung nach einem der Ansprüche 1 bis 3, dadurch gekennzeichnet, daß die Verbindung das Produkt der Reaktion des Säurechlorids

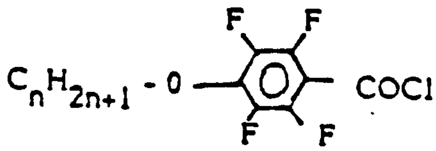

mit dem Phenol

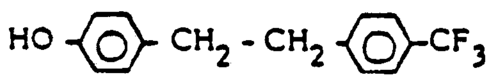

ist, die bei Umgebungstemperatur in Pyridin abläuft.

7. Verfahren nach Anspruch 6, dadurch gekennzeichnet, daß es folgende Verfahrensschritte umfaßt:

a) Herstellung des 4-Bromphenylacetylchlorids durch Einwirkung von Thionylchlorid auf die 4-Bromphenylessigsäure,

b) Synthese des 4-(4'-Bromphenylacetyl)-4'-methoxyphenyls durch FRIEDEL-CRAFT-Reaktion;

c) Reduktion des in der vorhergehenden Stufe erhaltenen Produkts durch eine modifizierte WOLF-KISHNER-Reaktion durch Einwirken von Hydrazin in basischem Milieu und in Anwesenheit von Diethylenglycol;

d) Trifluormethylierung des in der Stufe c) erhaltenen Produkts mit Jodtrifluormethan in Gegenwart von Kupfer bei 150°C in einem Autoklaven;

e) Demethylisierung des in der Stufe d) erhaltenen Produkts durch Verwendung von Jodtrimethylsilan als Mittel zum Aufspalten der Methylätherfunktion.

**Claims**

1. An organic compound presenting at least one mesomorphous phase of the smectic A type, characterized in that it corresponds to the general chemical formula:

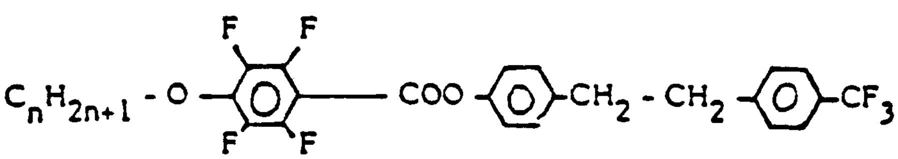

with 1 ⩽ n ⩽ 15.

2. An organic compound according to claim 1, characterized in that n = 8.

3. An organic compound according to claim 1, characterized in that n = 11.

4. A mixture of liquid cristals presenting at least one smectic A phase, characterized in that it comprises at least one organic compound according to any one of claims 1 to 3.

5. A mixture according to claim 4, characterized in that it comprises 66 % of 4-octyl-4'-cyanobiphenyl, 22,6 % 4-decyl-4'-cyanobiphenyl and 11,4 % of said organic compound with n = 8.

6. A method of preparing an organic compound according to any one of claims 1 to 3, characterized in that said compound is a product of the reaction of the acid chloride

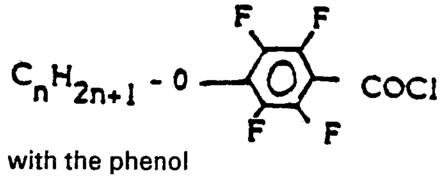

with the phenol

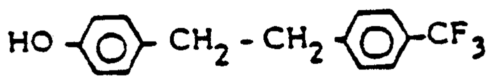

said reaction taking place at ambient temperature in pyridine.

7. A method according to claim 6, characterized in that it comprises the following steps:

a) formation of 4-bromophenylacetylchloride by treating 4-bromophenylacetic acid with thionylchloride;

b) synthese of 4-(4'-bromophenylacetyl)-4'-methoxyphenyl through the FRIEDEL-CRAFT reaction;

c) reduction of the product obtained in the preceding step through a modified WOLF-KISHNER-reaction by a treatment with hydrazine in a basic medium and in the presence of diethylenegylcol;

d) trifluoromethylation of the product obtained in step c by means of iodotrifluoromethane in the presence of copper at 150° C in an autoclave; and

e) demethylation of the product obtained in step d by means of iodotrimethylsilane as agent for splitting the methylether function.

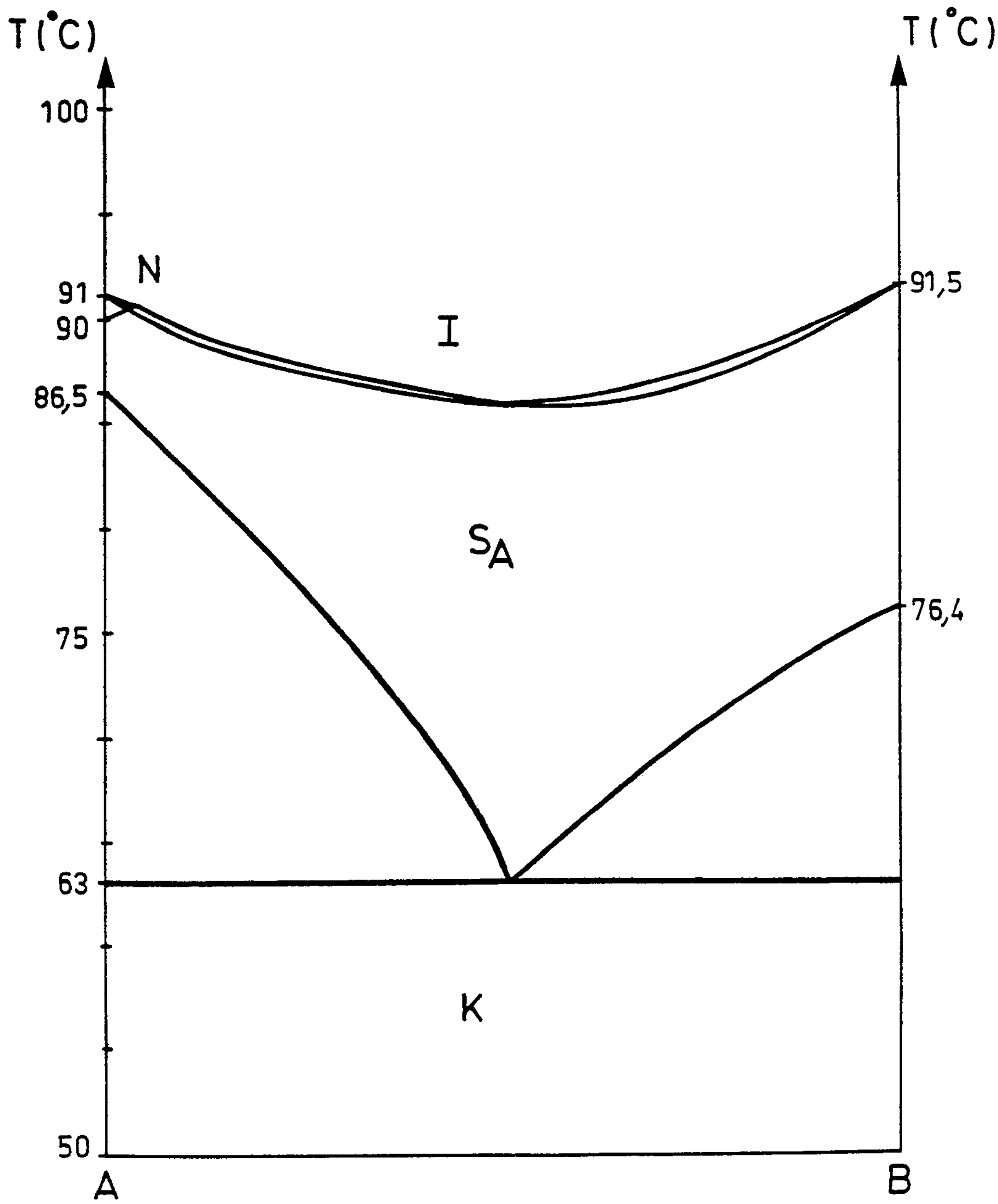
T (°C)
T (°C)
100
91
90
86,5
75
63
50
N
I
$S_A$
K
91,5
76,4
A
B